# EUROPÄISCHE PATENTSCHRIFT

(11) **EP 1 886 666 B1**
(45) Veröffentlichungstag und Bekanntmachung des Hinweises auf die Patenterteilung: **13.05.2009**
(21) Anmeldenummer: 07013433.3
(22) Anmeldetag: 10.07.2007
(51) Int. Cl.: A61K 9/00, C08J 3/21

(54) **Verfahren zur Herstellung eines Komposits aus Oligo- oder Polynucleotiden und hydrophoben biodegradierbaren Polymeren sowie nach dem Verfahren erhaltenes Komposit**
Method for manufacturing a composite made of oligo- or polynucleotide and hydrophobic biodegradable polymers and composite created according to this method
Procédé destiné à la fabrication d'un composite à partir d'oligonucléotides ou de polynucléotides et de polymères hydrophobes biodégradables tout comme composite obtenu par ce procédé

(30) Priorität: 07.08.2006 DE 102006038240
(43) Veröffentlichungstag der Anmeldung: 13.02.2008
(73) Patentinhaber: BIOTRONIK VI Patent AG, 6341 Baar (CH)
(72) Erfinder: Borck, Alexander, Dr., 91086 Aurachtal (DE); Diener, Tobias, Dr., 91058 Erlangen (DE)
(74) Vertreter: Lindner-Vogt, Karin L.

(56) Entgegenhaltungen:
- WO-A-01/45742
- WO-A-03/030941
- WO-A-20/05120453
- WO-A1-99/03454
- US-A1- 2002 156 047
- US-A1- 2003 049 320

## Beschreibung

Die Erfindung betrifft ein Verfahren zur Herstellung eines Komposits aus Oligo- oder Polynucleotiden und hydrophoben biodegradierbaren Polymeren, ein nach dem Verfahren erhaltenes Komposit sowie eine Verwendung des Komposits.

Oligo- und Polynucleotide werden im zunehmenden Maße als therapeutische Wirkstoffe eingesetzt. Eine parenterale Aufnahme der Wirkstoffe ist in der Regel nicht möglich; vielmehr muss eine interstitielle Verabreichung erfolgen. Ein Problempunkt der Entwicklung einer geeigneten Galenik ist die geringe Löslichkeit der Oligo- und Polynucleotide in hydrophoben biodegradierbaren Polymeren. Diese Polymere stellen jedoch für die Zwecke der interstitiellen Verabreichung besonders geeignete Trägersysteme dar. Aufgrund der hydrophoben Matrixeigenschaften ist nun zum Einen die Darstellung homogener Komposite, in denen die Nucleotide fein verteilt vorliegen, erschwert und zum Anderen sind derartige Komposite nicht ausreichend lagerstabil, denn es kommt zur Rekristallisation der zunächst in amorphen Modifikationen vorliegenden Nucleotide. Die Rekristallisation ändert das Freisetzungsverhalten der Nucleotide aus der biodegradierbaren Polymermatrix, so dass im Extremfall den Vorgaben der Therapie nicht mehr gefolgt werden kann.

Aufgabe der vorliegenden Erfindung ist es, die genannten Nachteile des Standes der Technik zu überwinden.

Nach einem ersten Aspekt der Erfindung wird diese Aufgabe durch das Verfahren zur Herstellung eines Komposits aus Oligo- oder Polynucleotiden und hydrophoben biodegradierbaren Polymeren nach Anspruch 1 gelöst. Das Verfahren umfasst die Schritte:
(i) Herstellen einer wässrigen Lösung aus einem Polyethylenglycol mit einem mittleren Molekulargewicht im Bereich von 600 bis 8.000 g/mol;
(ii) Zugabe einer wässrigen, gegebenenfalls gepufferten, Lösung von Oligo- oder Polynucleotiden zur wässrigen Lösung aus Schritt (i);
(iii) Einfrieren der wässrigen Lösung aus Schritt (ii) und anschließendes Gefriertrocknen der gefrorenen Lösung unter Erhalt eines gefriergetrockneten Substrats;
(iv) Bereitstellen einer Lösung des hydrophoben biodegradierbaren Polymers in einem organischen Lösungsmittel;
(v) Zugabe des nach Schritt (iii) erhaltenen Substrats zur Lösung des Polymers und
(vi) Entfernen des organischen Lösungsmittels unter Erhalt des Komposits.

Es hat sich gezeigt, dass nach dem erfindungsgemäßen Verfahren hergestellte Komposite wesentlich lagerstabiler sind und eine sehr homogene Verteilung der Nucleotide aufweisen. Offenbar bildet sich in den Schritten (i) bis (iii) ein Konjugat aus Polyethylenglycol und den Nucleotiden. Dieses Konjugat scheint auch in den weiteren Verfahrensschritten Stabilität zu besitzen und die gewünschten Effekte einer verbesserten Löslichkeit und Lagerstabilität mitzuprägen. Es hat sich gezeigt, dass Polyethylenglycol die Rolle eines Lösungsvermittlers wahrnehmen kann, so dass davon auszugehen ist, dass Polyethylenglykol-Moleküle im Konjugat die Nucleotide umhüllen. Das Konjugat trennt sich offenbar beim Lösen im organischen Lösungsmittel nicht wieder oder nur in einem geringen Ausmaß; vielmehr scheint ein thermodynamisch günstiger Zustand erreicht zu sein. Die wesentlich bessere Löslichkeit des Konjugats gegenüber den reinen Nucleotiden erlaubt nun die Herstellung hoch homogener Komposite. Diese Komposite besitzen zudem eine wesentlich höhere Lagerstabilität, offenbar aufgrund der stabilisierenden Effekte der Polyethylenmatrix.

Im Schritt (i) des Verfahrens wird eine wässrige Lösung aus einem Polyethylenglykol mit einem mittleren Molekulargewicht im Bereich von 600 bis 8.000 g/mol hergestellt.

Polyethylenglycol (PEG) ist eine Bezeichnung für zur Klasse der Polyether gehörende Polyalkylenglycole. Polyethylenglycole sind nichtionogene Verbindungen der allgemeinen Formel:

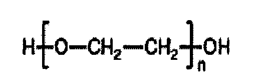

Polyethylenglycole werden technisch hergestellt durch anionische Ringöffnungspolymerisation von Ethylenoxid (Oxiran), meist in Gegenwart geringer Mengen Wasser (oder auch Natriummethylat oder Alkalihydroxid). Sie haben je nach Reaktionsführung Molmassen im Bereich von ca. 200 bis 5.000.000 g/mol, entsprechend Polymerisationsgraden Pn von ca. 5 bis > 100.000. Polyethylenglycole sind flüssige oder wachsartige bis feste Produkte, die sich in Wasser bis ca. 100°C und in vielen organischen Lösungsmitteln gut lösen. Polyethylenglycole sind physiologisch weitgehend innert und werden als toxikologisch unbedenklich eingestuft. Ihre biologische Abbaubarkeit ist stark Molmassen-abhängig; Produkte mit niedrigen Molmassen, z.B. 4.000 g/mol, werden bis zu 80% abgebaut. In der pharmazeutischen Technologie werden Polyethylenglycole als Lösungsvermittler, Weichmacher, Salbengrundlage, Emulgatoren, Schmier- und Gleitmittel in der Tablettenherstellung und als Bestandteil von Dragiersuspensionen verwendet.

Für die erfindungsgemäßen Zwecke werden Polyethylenglycole mit einem mittleren Molekulargewicht im Bereich von 600 bis 8.000 g/mol eingesetzt. Polyethylenglykole in dem genannten Molekulargewichtsbereich zeigen eine hinreichend hohe Wasserlöslichkeit. Polyethylenglykole mit einem Molekulargewicht von kleiner 600 g/mol haben einen ausgeprägten hydrophilen Charakter, so dass sie als Lösungsvermittler für die erfindungsgemäßen Zwecke nicht geeignet erscheinen. Möglicherweise ist bei relativ geringen Kettenlängen des Polyethylenglykols auch die Bildung eines Konjugats thermodynamisch ungünstiger. Polyethylenglykole mit einem Molekulargewicht von über 8.000 g/mol scheinen dagegen für die erfindungsgemäßen Zwecke einen zu stark hydrophoben Charakter zu besitzen, so dass die Bildung eines Konjugats mit den Nucleotiden erschwert ist. Möglicherweise ist auch die Kettenlänge für die Bildung eines solchen Konjugats zu groß.

Die wässrige Lösung aus Schritt (i) enthält Polyethylenglykol vorzugsweise in Konzentrationen von 0,1 bis 5 g/l Polyethylenglycol. Besonders bevorzugt liegt die Konzentration bei 0,5 bis 1 g/l. Es hat sich gezeigt, dass ein Arbeiten außerhalb der genannten Bereichsgrenzen offenbar die Bildung des Konjugats erschwert.

Im Schritt (ii) erfolgt die Zugabe einer wässrigen gegebenenfalls gepufferten Lösung von Oligo- oder Polynucleotiden zur wässrigen Lösung aus Schritt (i). Oligo- oder Polynucleotide sind in der Regel gut wasserlöslich und werden zur Stabilisierung in gepufferten Lösungen aufbewahrt. Die Lösung enthält vorzugsweise das Nucleotid in einer Konzentration im Bereich von 0,1 bis 7 µg/µL

Vorzugsweise wird ein Mengenverhältnis von Oligo- oder Polynucleotiden zum Polyethylenglykol derart festgelegt, dass Polyethylenglykol im 1,2 bis 100fachen gravimetrischen Überschuss vorliegt.

Oligonucleotide sind Polymerisate aus etwa 2 bis 10 Mono-Nucleotiden, die durch Phosphorsäurediester-Brücken verknüpft sind. Sie entstehen bei der Spaltung von Polynucleotiden und Nucleinsäuren oder durch Synthese, z. B. nach der Festphasen-Technik. Anwendung finden sie z. B. als Antisense-Inhibitoren der Genexpression. Polynucleotid ist eine Sammelbezeichnung für Polymere, die aus mehr als 10 über 3',5'-Phosphodiester-Brücken verknüpften Nucleotid-Einheiten aufgebaut sind. Zu den Polynucleotiden gehören vor allem die natürlich vorkommenden Nucleinsäuren, aber auch synthetische Produkte. Vorzugsweise liegen die Oligo- oder Polynucleotide als DNA-Fragmente, insbesondere dODN's vor. Das Oligo- oder Polynucleotid kann als Einzel- oder Doppelstrang vorliegen.

Im Schritt (iii) des Verfahrens wird die wässrige Lösung aus Schritt (ii) zunächst eingefroren. Anschließend wird die gefrorene Lösung gefriergetrocknet, wobei ein gefriergetrocknetes Substrat anfällt. Dieses Substrat kann eine watteartige Konsistenz besitzen.

Im Schritt (iv) des Verfahrens wird eine Lösung des hydrophoben biodegradierbaren Polymers in einem organischen Lösungsmittel bereitgestellt. Vorzugsweise ist das organische biodegradierbare Polymer ein Polylactid, insbesondere ein Poly-L-lactid.

Polylactide sind Polyester auf Basis von Milchsäure, aus deren Lactid sie durch Ringöffnungspolymerisation hergestellt werden können. Polylactid ist als Polyhydroxycarbonsäure biologisch abbaubar und wird unter anderem als resorbierbares chirurgisches Nahtmaterial und als Verkapselungsmaterial für Pharmaka eingesetzt.

Geeignete organische Lösungsmittel umfassen: Aceton, Ethanol, Ethylacetat, Acetylaceton, Hexafluoroisopropanol, THF und Dichlormethan.

Vorzugsweise ist das organische Lösungsmittel Chloroform.

Die Lösung enthält vorzugsweise das hydrophobe biodegradierbare Polymer in einer Konzentration im Bereich von 0,5 bis 7,5 g/Liter.

Im Schritt (v) erfolgt die Zugabe des nach Schritt (iii) erhaltenen Substrats zur Lösung des Polymers. Die Zugabe erfolgt unter Rühren der Lösung, um eine möglichst homogene Verteilung des sich lösenden Substrats zu erreichen. Vorzugsweise liegt, dass hydrophobe biodegradierbare Polymer im 1 bis 80fachen gravimetrischen Überschuss zu den Oligo- oder Polynucleotiden vor.

Im Schritt (vi) wird das organische Lösungsmittel unter Erhalt des gewünschten Komposits entfernt. In der Regel wird der Abzug des organischen Lösungsmittels unter vermindertem Druck bei Raumtemperatur erfolgen, um eine thermisch induzierte Metabolisierung der Nucleotide zu vermeiden.

Ein zweiter Aspekt der Erfindung richtet sich auf Komposite, die nach dem zuvor geschilderten Verfahren erhalten oder erhältlich sind.

Schließlich richtet sich ein dritter Aspekt der Erfindung auf die Verwendung eines solchen Komposits als Beschichtungsmaterial für ein medizinisches Implantat oder als Füllstoff einer Kavität eines medizinischen Implantats.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, Darmklammern, Gefäßclips, Prothesen im Bereich des Hart- und Weichgewebes und Ankerelemente für Elektroden, insbesondere von Schrittmachern oder Defibrillatoren.

Vorzugsweise ist das Implantat ein Stent. Stents herkömmlicher Bauart weisen eine filigrane Stützstruktur aus metallischen Streben auf, die zur Einbringung in den Körper zunächst in einem nicht expandierten Zustand vorliegt und die am Ort der Applikation dann in einen expandierten Zustand aufgeweitet wird. Aufgrund der nur geringen Größe der für eine Beschichtung geeigneten Flächen beziehungsweise Volumina von Kavitäten, müssen relativ hochkonzentrierte Komposite eingesetzt werden, um den therapeutischen Vorgaben am Ort der Implantation zu entsprechen. Ferner werden Stents in der Regel bevorratet, d. h. auch die Beschichtung/die Füllung muss über einen bestimmten Zeitraum lagerstabil sein. Mit Hilfe des erfindungsgemäßen Komposits lassen sich hochkonzentrierte nucleotidhaltige Matrizes realisieren, die zudem die notwendige Lagerstabilität besitzen.

Lösungen der folgenden Zusammensetzungen wurden hergestellt:
- Lösung 1:: Eine Lösung von 1 g Polyethylenglykol mit einem mittleren Molekulargewicht im Bereich von 4.000 bis 6.000 g/mol in 500 ml destilliertem Wasser.
- Lösung 2:: Eine Lösung eines dODN's mit einem Molekulargewicht von 10.000 g/mol in einer gepufferten wässrigen Lösung, enthaltend als Puffer.
- Lösung 3:: Eine Lösung von Poly-L-lactid 1,5 g mit einem mittleren Molekulargewicht im Bereich von 94.000 bis 700.000 g/mol in 200 ml Chloroform.

0,5 ml der Lösung 2 wurden bei Raumtemperatur und unter Rühren zu 500 ml der Lösung 1 zugesetzt. Hiernach wurde die erhaltene Lösung auf -25 °C abgekühlt und die erhaltene gefrorene Lösung mechanisch zerkleinert sowie unter folgenden Bedingungen gefriergetrocknet:

Die Gefriertrocknung geschah unter verminderten Druck (3 x 10⁻⁶ Bar) über 24 Stunden um sicherzustellen, dass das Wasser komplett entfernt wurde.

Das nach dem Gefriertrocknen erhaltene Substrat wurde unter Rühren und bei Raumtemperatur in 200 ml der Lösung 3 gegeben. Anschließend wurde das organische Lösungsmittel unter vermindertem Druck bei Raumtemperatur abgezogen.

## Patentansprüche

1. Verfahren zur Herstellung eines Komposits aus Oligo- oder Polynucleotiden und hydrophoben biodegradierbaren Polymeren, umfassend die Schritte:
(i) Herstellen einer wässrigen Lösung aus einem Polyethylenglycol mit einem mittleren Molekulargewicht im Bereich von 600 bis 8.000 g/mol;
(ii) Zugabe einer wässrigen, gegebenenfalls gepufferten Lösung von Oligo- oder Polynucleotiden zur wässrigen Lösung aus Schritt (i);
(iii) Einfrieren der wässrigen Lösung aus Schritt (ii) und anschließendes Gefriertrocknen der gefrorenen Lösung unter Erhalt eines gefriergetrockneten Substrats;
(iv) Bereitstellen einer Lösung des hydrophoben biodegradierbaren Polymers in einem organischen Lösungsmittel;
(v) Zugabe des nach Schritt (iii) erhaltenen Substrats zur Lösung des Polymers; und
(vi) Entfernen des organischen Lösungsmittels unter Erhalt des Komposits.

2. Verfahren nach Anspruch 1, bei dem die wässrige Lösung aus Schritt (i) Polyethylenglycol im 1,2 bis 100fachen gravimetrischen Überschuss zum Oligo- oder Polynucleotid enthält.

3. Verfahren nach Anspruch 1, bei dem die Oligo- oder Polynucleotide DNA-Fragmente, insbesondere dODN's, umfassen.

4. Verfahren nach Anspruch 1, bei dem das hydrophobe biodegradierbare Polymer ein Polylactid, insbesondere Poly-L-lactid ist.

5. Verfahren nach Anspruch 1, bei dem das organische Lösungsmittel aus Schritt (iv) Chloroform ist.

6. Komposit erhalten oder erhältlich nach einem Verfahren gemäß einem der Ansprüche 1 bis 5.

7. Verwendung eines Komposits nach Anspruch 6 als Beschichtungsmaterial für ein medizinisches Implantat oder als Füllstoff einer Kavität eines medizinischen Implantats.

## Claims

1. A method for producing a composite made of oligonucleotides or polynucleotides and hydrophobic biodegradable polymers, comprising the following steps:
(i) producing an aqueous solution of a polyethylene glycol having a mean molecular weight in the range from 600 to 8,000 g/mole;
(ii) adding an aqueous, possibly buffered solution of oligonucleotides or polynucleotides to the aqueous solution from step (i);
(iii) freezing the aqueous solution from step (ii) and subsequently freeze-drying the frozen solution to obtain a freeze-dried substrate;
(iv) providing a solution of the hydrophobic biodegradable polymer in an organic solvent;
(v) adding the substrate obtained according to step (iii) to the solution of the polymer; and
(vi) removing the organic solvent to obtain the composite.

2. The method according to claim 1, wherein the aqueous solution from step (i) contains polyethylene glycol in 1.2 to 100 fold gravimetric excess to the oligonucleotide or polynucleotide.

3. The method according to claim 1, wherein the oligonucleotides or polynucleotides comprise DNA fragments, in particular dODN.

4. The method according to claim 1, wherein the hydrophobic biodegradable polymer is a polylactide, in particular poly-L-lactide.

5. The method according to claim 1, wherein the organic solvent from step (iv) is chloroform.

6. A composite obtained or obtainable according to a method according to claim 1 to 5.

7. A use of a composite according to claim 6 as a coating material for a medical implant or as a filler of a cavity of a medical implant.

## Revendications

1. Procédé pour fabriquer un composite à base d'oligonucléotides ou de polynucléotides et de polymères biodégradables hydrophobes, comprenant les étapes suivantes :
(i) fabrication d'une solution aqueuse à base d'un glycol de polyéthylène avec un poids moléculaire moyen dans la plage de 600 à 8.000 g/mole ;
(ii) addition d'une solution aqueuse, éventuellement tamponnée, d'oligonucléotides ou de polynucléotides pour la solution aqueuse provenant de l'étape (i) ;
(iii) gel de la solution aqueuse provenant de l'étape (ii) suivi du séchage par congélation de la solution gelée avec obtention d'un substrat séché par congélation ;
(iv) mise à disposition d'une solution du polymère hydrophobe biodégradable dans un solvant organique ;
(v) addition du substrat obtenu après l'étape (iii) pour la solution du polymère ; et
(vi) enlèvement du solvant organique avec obtention du composite.

2. Procédé selon la revendication 1, dans lequel la solution aqueuse provenant de l'étape (i) contient du polyéthylèneglycol dans 1,2 à 100 fois l'excédent gravimétrique pour l'oligonucléotide ou le polynucléotide.

3. Procédé selon la revendication 1, dans lequel les oligonucléotides ou polynucléotides comprennent des fragments de DNA, en particulier des dODN's.

4. Procédé selon la revendication 1, dans lequel le polymère hydrophobe biodégradable est un polylactide, en particulier un poly-L-lactide.

5. Procédé selon la revendication 1, dans lequel le solvant organique provenant de l'étape (iv) est du chloroforme.

6. Composite obtenu ou réalisable selon un procédé conformément à l'une des revendications 1 à 5.

7. Utilisation d'un composite selon la revendication 6 comme matériau de revêtement pour un implant médical ou comme matière de charge d'une cavité d'un implant médical.
